Europäisches Patentamt

(19) European Patent Office

Office européen des brevets

(11) Publication number: **0 072 689**

A2

(12) **EUROPEAN PATENT APPLICATION**

(21) Application number: **82304303.9**

(22) Date of filing: **16.08.82**

(51) Int. Cl.³: **A 61 B 1/00**

(30) Priority: **19.08.81 JP 129666/81**

(43) Date of publication of application:
**23.02.83 Bulletin 83/8**

(84) Designated Contracting States:
**AT BE CH DE FR GB IT LI NL SE**

(71) Applicant: **OLYMPUS OPTICAL CO., LTD.**
**43-2, 2-chome, Hatagaya Shibuya-ku**
**Tokyo(JP)**

(72) Inventor: **Mizumoto, Morihide**
**Olympus Ryo 4-22-13 Ohwadacho**
**Hachioji-shi Tokyo(JP)**

(74) Representative: **Kennedy, Victor Kenneth et al,**
**G.F. REDFERN & CO. Marlborough Lodge 14 Farncombe**
**Road**
**Worthing West Sussex BN11 2BT(GB)**

(54) Endoscopes of the type provided with a sampling device.

(57) An endoscope with a sampling device which can positively and quickly take samples of deposits on the inner wall of a pipe or the like or of cells on the inner wall of a body cavity when inserted through a forceps channel (22) in the endoscope (11). For taking samples purely by a suction action within a pipe, or in a living body, samples cannot be easily obtained in some cases, as positivity force is lacking, and if it is necessary to sample a powder, simultaneous scraping and sucking actions are required to obtain the powder sample. Therefore, in an endoscope constructed in accordance with the present invention the sampling device is a hollow suction tube (22) which can be inserted through a channel (20) in the endoscope (11), and a scraping means (26) formed near an opening (27) at the tip of the suction tube (22), the tube (22) being connected at the rear hand grip to a suction means (24) to suck samples that are scraped off by the scraping means (26) so as to positively extract the samples.

**FIG.2**

EP 0 072 689 A2

## ENDOSCOPES OF THE TYPE PROVIDED WITH A SAMPLING DEVICE

The invention relates to endoscopes of the type provided with a sampling device which can positively take samples when inserted through a forceps channel formed in the endoscope.

Generally, in chemical plant, water disposing plant, or other such machinery, when a substance such as rust or any other impurity is contained in the liquid, be it water, oil or some solution, the substance tends to be deposited on the inner wall of any pipe or like channel transmitting the liquid, and the flow rate of the liquid will be reduced, not only reducing the production efficiency, but also possibly causing an accident or failure. Therefore, it is necessary to take and examine samples of deposits on the inner wall of such pipes or channels, and where necessary, to remove the deposits physically.

Similarly, in the case of a living body, it may be necessary to take samples of cells on the inner wall of a body passage to investigate the state within the living body.

When taking samples of the above described deposits or cells from any position that is hard to directly observe, an elongate insertion sheath of an endoscope is inserted, the optical image of the observed position is formed by an objective optical system arranged at the tip portion of the insertion sheath, and is observed via an observing optical system of the endoscope, by which the image can be observed from the rear through an eyepiece on the operating hand grip via image transmitting means, a sampling forceps may be inserted through a forceps channel provided in the endoscope, and a sample sucked out together with sucked air by suction means, such as a syringe provided on the hand grip.

With such a sampling device, cells or the like may be sampled merely by the suction action, but cannot be sampled in this manner in cases where a positive extraction is needed and, in case the object to be sampled is a powder, it must be simultaneously scraped and sucked if usable samples are to be obtained.

Further, problems are presented if the amount required to be sampled is large or if there are numerous parts from which samples are to be taken, as the sampling operation may become very difficult.

One object of the present invention is to provide an endoscope sampling device which can be positively relied upon to take samples in the difficulty circumstances described above, even in cases where the object to be sampled is a. powder.

According to one exemplary embodiment of the invention there is provided an endoscope of the type provided with a sampling device, characterised in that a sampling forceps inserted through a channel formed in an endoscope is provided with a suction tube having a hollow air path, and with scraping means formed near an opening communicating with said air path, said tube being connectable at its exterior, operating end with a suction means provided outside the endoscope.

The invention will now be described with reference to the drawings, in which:-

Figure 1 is a side view of a known endoscope with a sampling forceps, intended for use in the examination of living bodies;

Figure 2 is a side view of a first exemplary embodiment of the invention, for use in industrial applications;

Figure 3(a) is a sectional view showing essential details of the sampling device used in a first embodiment;

Figure 3(b) is a cross-section on line A-A' in Figure 3(a);

Figure 4(a) is a sectional view showing essential details of the sampling device in a second embodiment;

Figure 4(b) is a sectional view on the line B-B' in Figure 4(a); and

Figure 5 is a partly sectional view showing details of a suction device used in the second exemplary embodiment.

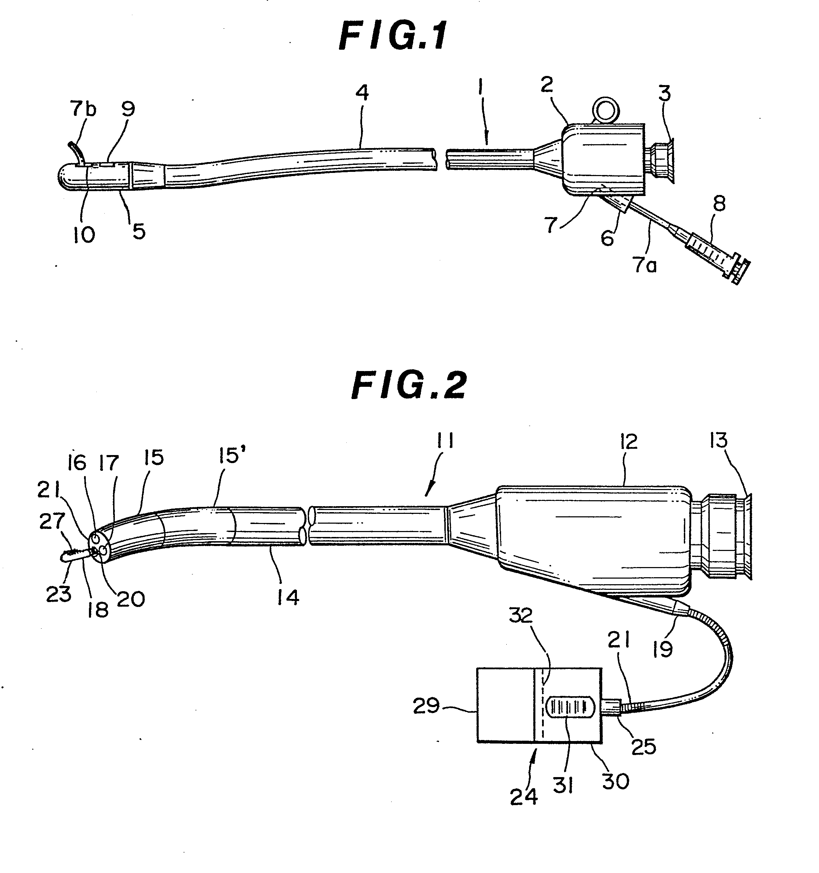

Prior to explaining the exemplary embodiments of the present invention, the conventional example of a known endoscope with a sampling device, disclosed in the gazette of Japanese utility model publication No. 7506/1972 will be described with reference to Figure 1.

An endoscope 1 for examination of living bodies comprises an operating hand grip 2, an eyepiece 3, a flexible tube 4 containing a light supply path and image transmitting means, and a tip portion 5. This endoscope 1 is provided with an internal forceps channel, through which a flexible sampling forceps can be removably inserted toward the tip portion 5 from a forceps inserting port 6 of the operating hand grip 2. The sampling forceps 7 is removably fitted with a suction device 8 such as a syringe connected to a port 7a on the operating hand grip, and has an end 7b that projects out of a slot 10 near an observing window 9 so as to reach the inner wall of a body cavity.

If the opening forming a sampling port for the tip 7b of the forceps 7 is closed completely, air cannot flow within the sampling forceps 7 and cells or the like will not be withdrawn to be sampled. Therefore, an air path is provided at the edge of the sampling port.

In case specimen cells or the like are to be sampled by using this sampling device, when the tip 7b of the sampling forceps 7 is butted against the inner wall of a body cavity in the part to be examined, while being observed through the observing window 9 of the endoscope 1, and air is sucked in by a suction instrument 8 through an air path provided inside the sampling forceps 7, the cells on the inner wall will be sucked into the sampling forceps 7. When the sampling ends, the foreceps 7 can be withdrawn from the endoscope 1, air can be fed to the forceps 7 from its tip 7a, and the cell sample drawn in to the suction port can be recovered.

In the sampling device of such structure, as cells are sampled merely by a suction action, they will not be sampled effectively in cases where more positive extraction means are necessary. If the object to be sampled is a powder, a scraping and suction action need to be simultaneously provided if the sampling is to be efficient.

Further, there has been a problem that, in cases where the amount required to be sampled is large, the sampling speed will be so low that the sampling will be very difficult.

The present invention is intended to solve these problems presented by the above mentioned conventional example.

Respective exemplary embodiments of the present invention will now be described with reference to the remaining figures.

Figure 2 shows an exemplary embodiment of an industrial endoscope 11 by way of example, formed by an operating hand grip 12, an eyepiece 13, a flexible insertion sheath 14, a tip portion 15 and a curved section 15' provided between the tip portion 15 and the insertion sheath 14.

A light-projecting end face 16 provides an illuminating light transmitted from the operating hand grip 12, and is formed together with an observing window 17 on the end surface of the tip portion 15. An observing optical system is arranged at the inner part of this window 17 and extends to the eyepiece 13. Further, a forceps channel 20 is internally provided so that sampling forceps 18, as illustrated, may be inserted through the endoscope from a forceps inserting port 19 provided in the operating hand grip 12.

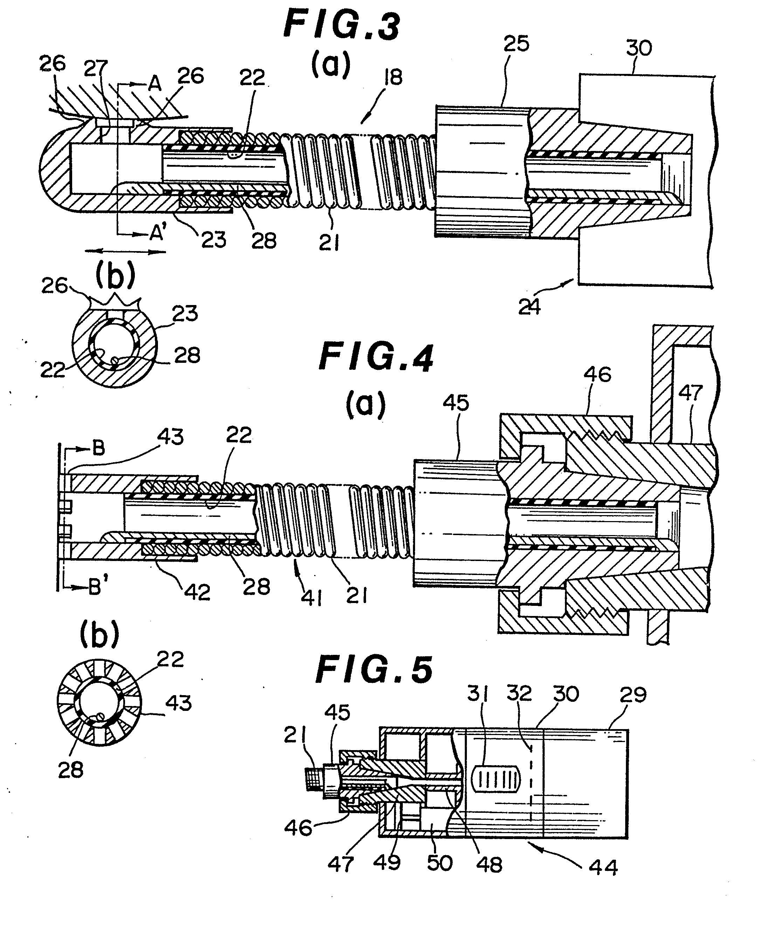

As shown in Figure 3, the sampling forceps 18 is formed by a highly flexible spiral wire tube 21 surrounding a flexible suction tube 22, a tip 23 at one end and a coupling connector 25 to a suction device 24, as shown in Figure 2, at the hand grip end. In the tip 23, a set of scraping teeth 26 are provided as scraping means for sampling, projecting so as to be opposed to each other forward and rearward (in the axial direction of the suction tube 22), three such teeth being shown in this embodiment. An opening 27 provided near the middle of the set of teeth communicates with an air path formed inside the suction tube 22.

The scraping teeth 26 provided to project in mutually opposed fashion are so formed as to sharply project at the peripheral ends of the opening 27 and to project in tapered manner to the outside so that, when the projecting parts of these scraping teeth 26 are butted against the inner wall of an object and are moved forwardly and rearwardly, the inside projecting surface peripheral end parts will scrape off the required samples from the inner wall surface of the cavity into which the endoscope is inserted.

A wire 28 is provided through the suction tube 22 between the tip 23 and connector 25 in order to increase the strength and to facilitate a strongly butting action of the teeth against the inner wall surface of a pipe or the like.

The suction device 24 is connected by pressing and fitting a tapered end of the connector 25 to a suction means 29 leading to a containing chamber 30. The suction means 29 acts to suck in air, and so suck in samples freed by the teeth. The containing chamber 30 is provided with an indicator so as to permit observation of the samples and the amount thereof collected in this chamber. A filter 32 is provided in the chamber 30, so that the samples may not enter the suction means 29.

When taking samples by using this embodiment, an illuminating light from the end face 16 is taken in through the observing window 17 of the endoscope and the scraping teeth 26 formed in the tip 23 of the sampling forceps 18 are butted against the inner wall of the object while being observed, as schematically shown in Figure 3. By manually operating the flexible spiral wire tube 21 that extends between the forceps inserting port 19 and the connector 25, the above mentioned scraping teeth 26 can be reciprocated forwardly and rearwardly, as shown by arrows, to scrape a sample from the surface of the object to be examined, and by operating the suction device 24, air is drawn in through the opening 27 to take the scraped sample via the opening 27 into the containing chamber 30 through the suction tube 22.

Therefore, when the operator of this device sees the sampled amount indicator 31 that is provided within the containing chamber 30, he will know the sampled amount.  As the sample material is sucked into the containing chamber 30, many samples can be taken.

In the above described embodiment, the means of moving the scraping means forwardly and rearwardly is operated manually, but can be operated by a driving motor or the like, if required.

In the second exemplary embodiment, shown in Figure 4, the tip 42 of the sampling forceps 41 is formed with a plurality of scraping teeth 43 annularly disposed around the cylindrical end face so as to be mutually opposed, with an opening inside.  The connector 45 connecting the sampling forceps 41 with the suction device 44 is driven so as to rotate the sampling forceps 41 around  its central axis to rotate and drive these scraping teeth 43.

Thus, the sampling forceps 41 provided with an air path inside the suction tube 22 is connected with the connector 45 provided with an inserting port connected with this air path.  This connector 45 is formed to be tapered at the tip, as shown in Figure 4 for example, so as to be fitted in and connected, and is coupled to the suction device 44 that is provided with a connector receiver 47 so as to be removably fitted with the connector receiver 47 by a connecting ring 46 that is provided with a screw thread and engages with this connector 45.

The suction device 44 is further provided with a rotating and driving means as shown in Figure 5, the substantially cylindrical connector receiver 47 being formed with a tapered inner surface connected with the containing chamber 30 forming part of the suction device 44 so as to form an air path via a connecting pipe 48. This connector receiver 47 is pressed in contact with the annular outer peripheral part of a disk-shaped friction member 49 which is connected with the rotary shaft of a motor 50 that is contained outside the above mentioned connecting pipe 48, so that when the friction member 49 is rotated by the motor 50 the connector receiver 47 is rotated to rotate the sampling forceps 41 and the scraping teeth 43 in its tip 42.

Rotation may be achieved by using gears instead of the friction member 49. In the suction device 44, a filter 32 is fitted in the containing chamber 30 provided with the indicator 31, so that both the sampled amount and the samples themselves can be observed, as in the first described embodiment, and a suction means 29 is provided after the filter.

According to the thus formed second embodiment, as shown in Figure 4, when the scraping teeth 43 formed in the tip part 42 of the sampling forceps 41 are manipulated to butt against an object, the motor 50 is rotated and the action of the suction device 44 actuated, so that when the scraping teeth 43 rotate to scrape a sample off the surface of the inner wall of the object that is to be examined, this scraped sample will be sucked via the suction tube 22 together with air from the inside opening, and samples will be fed to the containing chamber 30, through the connector 45 and the connecting pipe 48.

Therefore, as the sampled amount can be observed by observing the indicator 31 provided in the containing chamber 30, the sample in any desired part can be taken easily, quickly and positively.

In this exemplary embodiment, the scraping means can be rotated manually instead of using the motor, and a manual suction device 24 may be used.

The scraping means of this invention is not limited to the above described configuration of scraping teeth 26 and 43. For example, a plurality of the scraping teeth of the shapes described in the first embodiment, and associated openings, may be provided in the rotary axial direction on the cylindrical outer periphery, so as to be rotated and driven, or rotated with a reciprocating action. This device can be used not only to take industrial samples as described above, but also to scrape, suck and remove unnecessary deposits off a surface to clean the internal surface of a pipe, for example. In such applications the device may be made larger to increase its efficiency.

It will be apparent that differing working modes in a wide range can be formed without deviating from the spirit and scope of the present invention, as defined in the appended claims.

For medical applications the constructional details may be varied to reduce the overall cross-section, but the means for sampling can remain combined with an endoscope to facilitate diagnostic or surgical processes.

CLAIMS:-

1.    An endoscope of the type provided with a sampling device, characterised in that a sampling forceps inserted through a channel formed in an endoscope is provided with a suction tube having a hollow air path, and with scraping means formed near an opening communicating with said air path, said tube being connectable at its exterior, operating end with a suction means provided outside the endoscope.

2.    An endoscope according to Claim 1, characterised in that said scraping means is moved forwardly and rearwardly manually, or by a driving device.

3.    An endoscope according to Claim 1, characterised in that means are provided by which said scraping means is rotated or reciprocated and rotated, manually or by a driving device.

4.    An endoscope according to Claim 1, characterised in that said scraping means is provided with projecting teeth that have a steep edge adjacent the sides of a central opening.

5.    An endoscope according to Claim 1, characterised in that said scraping means has a plurality of wedge-shaped blades formed in a peripheral end part and arranged in mutually opposed fashion.

6.    An endoscope according to Claim 1, characterised in that said suction means is formed of a suction device operative to suck samples into a containing chamber provided with a filter passing sucked gases and an indicator indicating suction effectiveness and contained sampled amounts.

7.    An endoscope according to Claim 1, <u>characterised</u> <u>in that</u> said scraping means and suction means operate simultaneously.

**FIG.1**

**FIG.2**

0072689

# FIG.3
## (a)

## (b)

# FIG.4
## (a)

## (b)

# FIG.5